# EUROPEAN PATENT SPECIFICATION

(11) **EP 1 825 835 B1**
(45) Date of publication and mention of the grant of the patent: **22.04.2009**
(21) Application number: 07250764.3
(22) Date of filing: 23.02.2007
(51) Int. Cl.: A61F 5/44

(54) **Urine collection system with needleless sampling port**
Urinsammelsystem mit nadellosem Probenahmeport
Système de collecte d'urine avec port de prélèvement sans aiguille

(30) Priority: 24.02.2006 US 362656
(43) Date of publication of application: 29.08.2007
(73) Proprietor: Tyco Healthcare Group LP, Mansfield, MA 02048 (US)
(72) Inventor: Salvadori, Lawrence, San Diego, California 92127 (US); Tully, Stephen J., Massachusetts 02141 (US)
(74) Representative: Gray, James

(56) References cited:
- GB-A- 2 011 523
- GB-A- 2 322 079
- US-A- 4 312 352

## Description

### BACKGROUND

### 1. Technical Field

The present disclosure relates generally to fluid collection systems. More specifically, the present disclosure relates to fluid collection systems including improved venting structure and means for obtaining a fresh sample of fluid from the system without contaminating fluid within the system or risking injury to medical personnel.

### 2. Background of Related Art

Urine collection systems for collecting urine from a catheterized patient are well known in the art. Such systems typically include a drain tube having a first end connected to a urinary catheter of a catheterized patient and a second end connected to a urine collection bag. The urine collection bag includes an outlet port for draining fluid from the bag.

It is sometimes desirable in fluid collection systems to access fluid in the system to obtain a sample for laboratory analysis. In known collection systems, as for example disclosed in GB 2322079, samples have been extracted using syringes or hypodermic needles which were inserted through walls of the drain tubing. Samples have also been obtained by piercing a rubber port on the collection bag itself or by draining fluid from the collection bag through the outlet port. Such sampling techniques expose medical personnel to potential needlestick injury and to urine contact. Moreover, such sampling techniques run the risk of contamination of fluid within the collection system, thus exposing a patient to potential infections.

When obtaining a urine sample from a urine collection system, it is desirable to take the most recent sample from the collection system. Old or stale samples can become contaminated rendering the urine sample unfit for analysis and subjecting medical personnel to contamination.

Urine collection systems including collection bags having a vent or vents formed therein are also well known. Such vents allow air to enter or exit the collection bag during emptying or filling of the collection bag. Although vents in the collection bag are somewhat effective, emptying of the collection bag can still effect siphoning of fluid from a patients bladder.

Accordingly, it would be desirable to provide a fluid collection system which facilitates the collection of fresh fluid samples from a collection system while minimizing the risk of contamination or injury of medical personnel and/or contamination of fluid within the collection system. It would also be desirable to provide a fluid collection system having more effective venting to prevent siphoning of fluid from a patient's bladder.

### SUMMARY

A urine collection system is provided which includes a fluid collection bag defining a fluid reservoir, a drain tube, and a sampling port. The collection bag includes a fluid inlet in fluid communication with one end of the drain tube and a fluid outlet. The drain tube has a second end adapted to be in fluid communication with a urinary catheter. The sampling port is supported in fluid communication with the drain tube upstream of the fluid inlet of the collection bag.

In one embodiment, the sampling port is adapted to engage a luer-lock or slip-tip type syringe. In another embodiment, the sampling port includes a flexible pierceable member which is self-sealing after use.

The urine collection system can also include an in-line vent located upstream from the collection bag. The in-line vent limits siphoning of a patient's bladder during emptying of the urine collection bag.

In another embodiment, the urine collection system includes a support member for hanging the urine collection bag on a support structure, e.g., bedframe. The support member includes a central body portion and at least one hook portion. The central body portion has first and second vertically spaced mounting structures for securing the support member to a urine collection bag. The first mounting structure facilitates securement of the support member to the urine collection bag at a first vertical position in relation to the bag and the second mounting structure facilitates securement of the support member to the urine collection bag at a second vertical position in relation to the bag different from the first vertical position. The first and second mounting structures can include first and second openings dimensioned to receive a pin or dowel. The urine collection bag would also include such an opening dimensioned to receive the pin or dowel.

In one embodiment, the at least one hook portion includes first and second hook portions depending from opposite sides of the central body portion. Each of the hook portions can be pivotally secured to the central body portion such as with a living hinge to enable adjustment of the hook portions. Adjustable hook portions allow the support member/collection bag assembly to be more easily hung on a support structure.

### Brief Description Of The Drawings

Various embodiments of the presently disclosed fluid collection system are disclosed herein with reference to the drawings, wherein:
FIG. 1 is a side perspective view of one embodiment of the presently disclosed fluid collection system;
FIG. 1A is a side perspective view with parts separated of the drain tube, sampling port and in-line vent of the collection system shown in FIG. 1;
FIG. 2 is a front view of a sampling port of the fluid collection system shown in FIG. 1;
FIG. 3 is a side view of the sampling port shown in FIG. 2;
FIG. 4 is a side perspective view of the sampling port shown in FIG. 3;
FIG. 5 is a side cross-sectional view of the sampling port shown in FIG. 4;
FIG. 6 is a side perspective view of an alternative embodiment of the presently disclosed sampling port;
FIG. 7 is a side cross-sectional view of the sampling port shown in FIG. 6;
FIG 8 is a front perspective view of a hanger support member of the fluid collection system shown in FIG. 1; and
FIG. 9 is a rear perspective view of the hanger support member shown in FIG. 8 with the support hooks folded ninety-degrees.

### DETAILED DESCRIPTION OF EMBODIMENTS

Embodiments of the presently disclosed fluid collection system will now be described in detail with reference to the drawings wherein like reference numerals designate identical or corresponding elements in each of the several views:

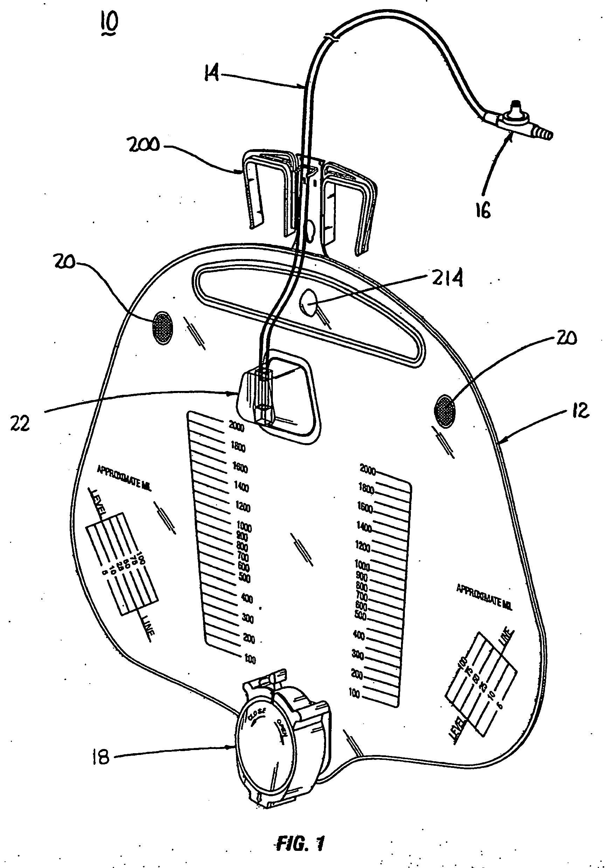

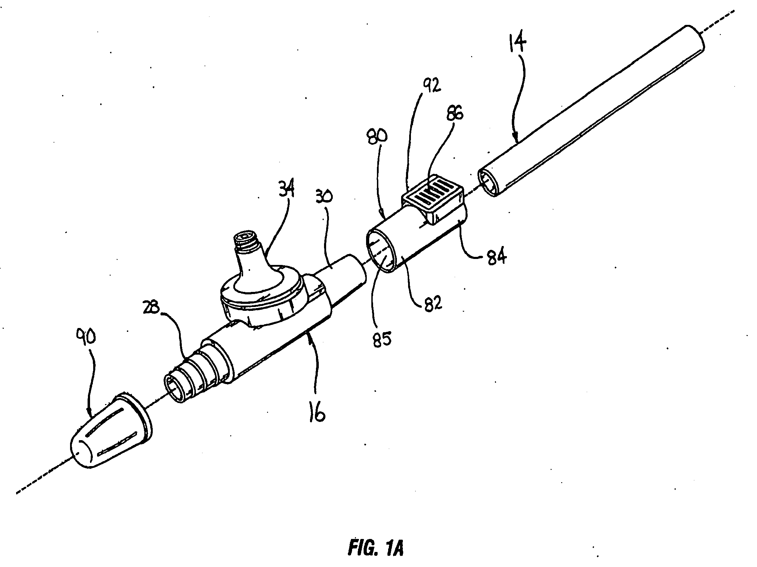

Referring to FIG. 1, fluid collection system 10 includes a fluid collection bag 12, a drain tube 14, a sampling port 16 connected to a first end 14a of drain tube 14 and a discharge valve 18. Collection bag 12 has at least one vent opening 20 for allowing air into and out of collection bag 12. An anti-reflux valve 22 is positioned at a second end 14b of drain tube 14. Anti-reflux valve 22 allows fluid to flow from drain tube 14 into collection bag 12 but restricts flow from collection bag 12 back into drain tube 14. Anti-reflux valve 22 can be secured directly to collection bag 12 using any known fastening technique, e.g., welding, adhesives, etc.

Fluid collection system 10 is used to collect fluid from a catheterized patient. The fluid flows via a transfer tube (not shown), through sampling port 16 and into drain tube 14. Fluid in tube 14 enters collection bag 12 through anti-reflux valve 22 where it collects in collection bag 12. Discharge valve 18 is operable in a known manner to selectively drain the fluid from collection bag 12.

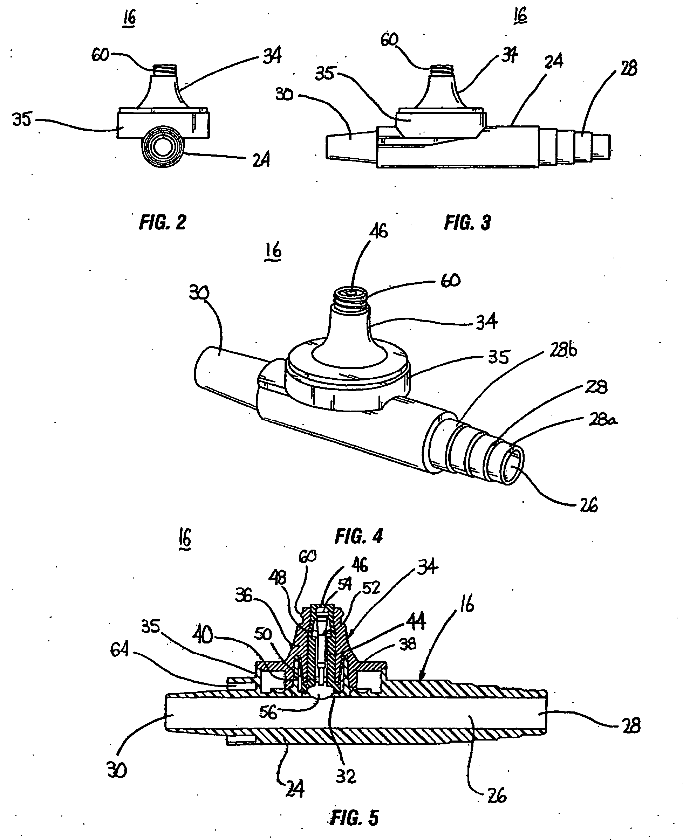

Referring to FIGS. 2-5, sampling port 16 includes a substantially rigid body portion 24 defining a longitudinal channel 26 having an inlet end 28, an outlet end 30, and a transverse opening 32 (FIG. 5). A valve assembly 34 is supported within a cup structure 35 formed on body portion 24. Valve assembly 34 includes an outer valve housing 36 which is supported within cup structure 35 and defines an annular recess 38 dimensioned to receive an annular inner valve housing 40 (FIG. 5). Inner valve housing 40 supports outer valve housing 36 above transverse opening 32 and can be formed integrally with body portion 24 or formed separately therefrom and secured thereto. Valve assembly 34 can be secured within cup structure 35 using any known fastening technique, e.g., adhesives RF or ultrasonic welding, etc. Outer valve housing 36 defines a channel 42 which is substantially orthogonal to channel 26. A flexible gland 44 which can be formed from silicone is positioned within channel 42 and defines an inlet 46, a throughbore 48 and an outlet 50 which communicates with opening 32 of body portion 24. A valve stem 52 is supported in throughbore 48 and functions to regulate flow through valve assembly 34. Valve stem 52 includes an upper valve member 54 supported adjacent inlet 46 within gland 44 and a lower valve member 56 positioned adjacent outlet 50. In its unbiased position, gland 44 maintains valve stem 52 in a position to seal inlet 46 and outlet 50 of throughbore 48 to prevent flow through sampling port 16.

Top valve housing 36 includes an outer surface having a thread 60 configured to releasably engage a luer lock or slip-tip type syringe (not shown). In use, when a slip tip or luer lock type syringe is attached to, i.e., secured onto thread 60, a tip of the syringe (not shown), enters inlet 46 of valve 34 and urges valve stem 52 downwardly against the bias of gland 44 to open valve 34. In the open position, fluid can be withdrawn by a syringe through opening 32 in body portion 24 of sampling port 16, such that fluid flows around valve stem 52 and exits opening 46 into the syringe. By withdrawing fluid from collection system 10 upstream from collection bag 12, a less stale, uncontaminated sample can be obtained.

As illustrated in FIGS. 3 and 4, inlet end 28 includes a stepped portion which has a distal end 28a of smaller diameter than its proximal end 28b. The stepped portion is configured to engage a transfer tube (not shown) which is connected to a catheter positioned within the bladder of a patient. Outlet end 30 includes a frusta-conical portion which is configured to receive one end of flexible drain tube 14 or a vent 80 (FIG. 1A) as will be discussed below. An annular recess 64 (FIG. 5) is provided about frusto-conical portion 30 for receiving the end of drain tube 14 or vent 80. It is envisioned other attaching techniques may be used to secure sampling port 16 between drain tube 14 and the transfer tube, e.g., clamps, adhesives, etc.

A valve such as valve 34 of sampling port 16 is available from NP Medical, Inc., a division of Nypro, Inc., of Clinton, Ma. It is envisioned that other valves which function in a similar manner may also be incorporated into body portion 24 of sampling port 16.

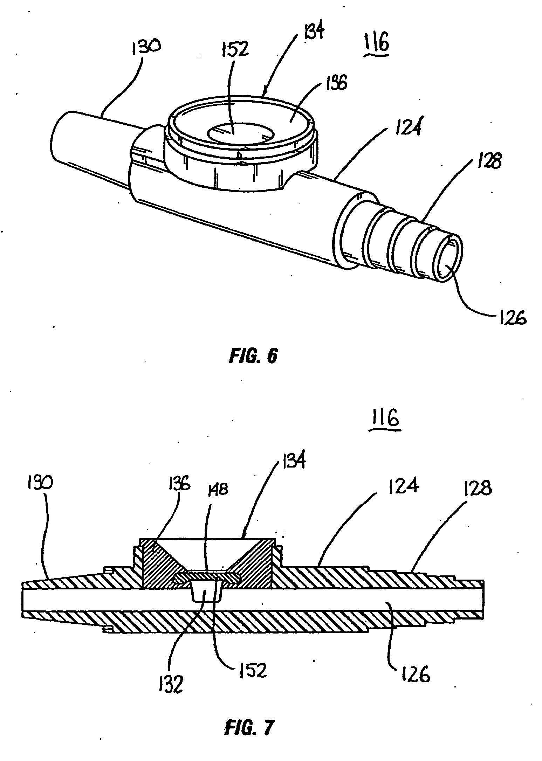

FIGS. 6 and 7 illustrate an alternate embodiment of the presently disclosed sampling port shown generally as 116. Sampling port 116 includes a body portion 124 defining a longitudinal channel 126 having an inlet end 128, an outlet end 130 and a transverse opening 132 (FIG. 7). Body portion 124 defines a recess or cup structure 133 configured to receive an access member 134. Although recess 133 is illustrated as being circular, other configurations are envisioned, e.g., square, rectangular, etc. Access member 134 includes a housing 136 defining a transverse throughbore 148 which is aligned with opening 132. A pierceable, flexible sealing member 152 is supported within housing 136 to seal throughbore 148. Sealing member 152 is formed from a material which can be pierced with the needle of a syringe to access fluid within sampling port 116 and will seal upon itself when the needle of the syringe is removed from sealing member 152.

Inlet end 128 and outlet end 130 of body portion 124 are substantially as described above with respect to ends 26 and 28 of body portion 24 of sampling port 16 and will not be described in further detail herein. In use, inlet end 128 of sampling port 116 is connected to a transfer tube (not shown) which is connected to a urinary catheter of a catheterized patient and outlet end 130 is connected to one end of drain tube 14 (FIG. 1) or to in-line vent 80 as will be described below, such that urine from the patient flows through the sampling port and the drain tube to collection bag 12. Next, a clinician or medical personnel pierces sealing member 152 with a needle of a syringe (not shown) and fluid is withdrawn from sampling port 116 upstream of collection bag 12. Thereafter, the needle of the syringe is withdrawn from sealing member 152 and sealing member 152 seals the piercing.

Referring to FIG. 1A, an in-line vent 80 can be provided between the catheterized patient (not shown) and collection bag 12. In one embodiment, in-line vent 80 has a first end 82 configured to engage outlet end 30 of sampling port 16 and a second end 84 configured to engage drain tube 14. In-line vent 80 includes a housing 92 defining a longitudinal throughbore 85 to allow fluid to flow through the vent housing 92. Vent housing 92 supports a venting structure 86 which allows air to enter the system to prevent siphoning of fluid from a patient's bladder during emptying of collection bag 12. Vent 80 also minimizes back pressure in the system especially when drain 14 is hung below bag 12. In one embodiment, venting structure 86 includes an oleophobic expanded PTFE membrane and housing 82 is formed from PVC. The oleophobic membrane can be a gortex^{™} material. Alternately, other materials be used to construct the membrane. As illustrated, a cap 90 can be provided to cover end 28 of sampling port 16 prior to attachment of sampling port 16 to a catheterized patient.

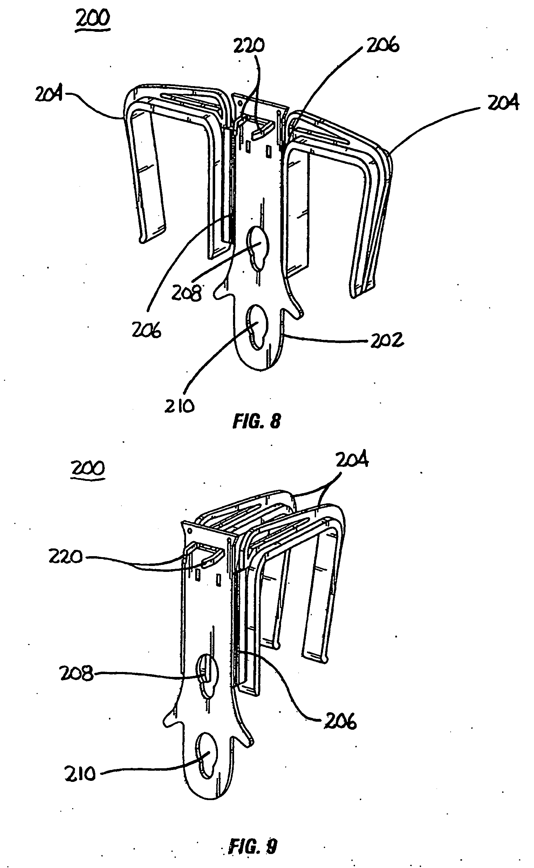

Referring to FIGS. 1, 8 and 9, fluid collection system 10 also includes a support member 200 for supporting collection bag 12 on support structure, e.g., bedframe (not shown). Support member 200 includes a central body portion 202 and at least one depending hanger or hook portion 204. In one embodiment, the at least one depending hook portion 204 includes a pair of hook portions 204 which extend outwardly from opposite sides of central body portion 202. Each hook portion 204 is pivotally connected to central body portion 202. Hook portions 204 can be pivotally connected to central body portion 202 by a living hinge 206. Alternately, other pivot structure can be used to secure hook portions 204 to central body portion 202, e.g., pivot pins. The pivot structure facilitates repositioning of hook portions 204 in relation to central body portion 202 to more easily and securely attach a collection bag 12 (FIG. 1) to support structure (not shown).

Central body portion 202 includes upper and lower mounting structure for securing support member 200 to collection bag 12 at vertically spaced positions. In one embodiment, upper and lower mounting structure includes an upper mounting opening 208 and a lower mounting opening 210. Mounting openings 208 and 210 are dimensioned to receive a securement dowel or pin 214 (FIG. 1) for securing support member 200 to collection bag 12. Pin 214 is positioned through an opening (not shown) in collection bag 12 and one of openings 208 and 210 to selectively secure support member 200 at one of two vertically spaced locations in relation to collection bag 12. By providing upper and lower mounting openings 208 and 210 in support member, the height of the collection bag in relation to the support member 200 can be selectively changed to thereby vary the height of the collection bag on the support structure. Since fluid collection system 10 is a gravity flow system, openings 208 and 210 allow for the head pressure of the system to be changed by changing the vertical positioning of collection bag 12 in relation to support member 200.

Central body portion 202 also includes a pair of spring arms 220. Spring arms 220 extend outwardly from central body portion 202 of support member 200 and are positioned to releasably engage drain tube 14. Spring arms 220 minimize the likelihood of drain tube 14 becoming twisted or kinked. Alternately, other tube support structure may be employed.

Drain tube 14 and collection bag 12 are formed from a flexible material or materials, e.g., polyvinylchloride ("PVC"). In one embodiment, the collection bag and drain tube are formed of a material having a durometer of between about 60 and about 100 and preferably about 78. By constructing the collection bag and/or the drain tube of a higher durometer material than typically employed, kinking of the bag and/or tube is minimized.

It will be understood that various modifications may be made to the embodiments disclosed herein. For example, the exact positioning of in-line vent and/or sampling port upstream of the collection bag can be altered and need not be exactly as shown. Further, although shown with two vertically spaced mounting structures, e.g., openings, the support member may include multiple vertically spaced mounting structures, e.g., 3 or more. Therefore, the above description should not be construed as limiting, but merely as exemplifications of preferred embodiments. Those skilled in the art will envision other modifications within the scope of the claims appended hereto.

## Claims

1. A urine collection system comprising:
a collection bag (12) defining a fluid reservoir and including a fluid inlet and a fluid outlet; and a support member (200), **characterized in that** said
support member including a central body portion having first (208) and second (210) vertically spaced mounting structures, each spaced mounting structure configured to facilitate securement of the support member to the collection bag, wherein the first spaced mounting structure facilitates securement of the collection bag to the support member to support the collection bag at a first vertical position and the second spaced mounting structure facilitates securement of the collection bag to the support member to support the collection bag at a second vertical position different than the first vertical position.

2. A urine collection system according to Claim 1, wherein the first and second spaced mounting structures include first and second openings formed in the central body portion dimensioned to receive a dowel (214).

3. A urine collection system according to Claim 1, further including a drain tube (14) in fluid communication with the fluid inlet; and
a sampling port (16) supported in fluid communication with the drain tube upstream of the fluid inlet.

4. A urine collection system according to Claim 3, wherein the sampling port includes an inlet end (28) adapted to be in fluid communication with a urinary catheter and an outlet end (30) adapted to be in fluid communication with the drain tube.

5. A urine collection system according to Claim 4, wherein the sampling port is adapted to engage a slip-tip or luer-lock type syringe.

6. A urine collection system according to Claim 4, wherein the sampling port includes a flexible pierceable sealing member (152).

7. A urine collection system according to Claim 3, further including an in-line vent (80) positioned in fluid communication with the drain tube upstream of the fluid inlet of the collection bag.

## Patentansprüche

1. Urinsammelsystem, umfassend:
einen Sammelbeutel (12), der einen Fluidbehälter definiert und einen Fluideinlass und einen Fluidauslass sowie ein Aufhängungselement (200) aufweist, **dadurch gekennzeichnet, dass**
das Aufhängungselement einen zentralen Körperteil mit einer ersten (208) und einer zweiten (210) senkrecht beabstandeten Befestigungsstruktur aufweist, wobei jede beabstandete Befestigungsstruktur dazu konfiguriert ist, eine Befestigung des Aufhängungselements am Sammelbeutel zu ermöglichen, wobei die erste beabstandete Befestigungsstruktur eine Befestigung des Sammelbeutels am Aufhängungselement ermöglicht, um den Sammelbeutel an einer ersten senkrechten Position aufzuhängen, und
die zweite beabstandete Befestigungsstruktur eine Befestigung des Sammelbeutels am Aufhängungselement ermöglicht, um den Sammelbeutel an einer zweiten senkrechten Position, die sich von der ersten senkrechten Position unterscheidet, aufzuhängen.

2. Urinsammelsystem nach Anspruch 1, wobei die erste und die zweite beabstandete Befestigungsstruktur eine erste und eine zweite Öffnung aufweisen, die im zentralen Körperteil ausgebildet sind, die so dimensioniert sind, dass sie einen Stift (214) aufnehmen.

3. Urinsammelsystem nach Anspruch 1, ferner mit einem Ablaufschlauch (14) in Fluidkommunikation mit dem Fluideinlass; und
einem Probenentnahmeanschluss (16), der in Fluidkommunikation mit dem Ablaufschlauch dem Fluideinlass in Strömungsrichtung vorgeschaltet angeordnet ist.

4. Urinsammelsystem nach Anspruch 3, wobei der Probenentnahmeanschluss ein Einlassende (28), das dazu ausgelegt ist, mit einem Urinkatheter in Fluidkommunikation zu sein, sowie ein Auslassende (30) aufweist, das dazu ausgelegt ist, mit dem Ablaufschlauch in Fluidkommunikation zu sein.

5. Urinsammelsystem nach Anspruch 4, wobei der Probenentnahmeanschluss dazu ausgelegt ist, mit einer Slip-Tip- oder Luer-Lock-Spritze in Eingriff zu kommen.

6. Urinsammelsystem nach Anspruch 4, wobei der Probenentnahmeanschluss ein flexibles durchstechbares Dichtungselement (152) aufweist.

7. Urinsammelsystem nach Anspruch 3, ferner mit einer Leitungslüftung (80), die in Fluidkommunikation mit dem Ablaufschlauch dem Fluideinlass des Sammelbeutels in Strömungsrichtung vorgeschaltet angeordnet ist.

## Revendications

1. Système de collecte d'urine comportant :
une poche de collecte (12) définissant un réservoir de fluide et présentant une entrée de fluide et une sortie de fluide ; et
un élément de support (200), **caractérisé en ce que** ledit élément de support comprend une partie de corps centrale ayant des première (208) et seconde (210) structures de montage espacées verticalement, chaque structure de montage espacée étant configurée de façon à faciliter la fixation de l'élément de support à la poche de collecte, dans lequel la première structure espacée de montage facilite la fixation de la poche de collecte à l'élément de support de façon à supporter la poche de collecte dans une première position verticale et la seconde structure espacée de montage facilite la fixation de la poche de collecte à l'élément de support de façon à supporter la poche de collecte dans une seconde position verticale différente de la première position verticale.

2. Système de collecte d'urine selon la revendication 1, dans lequel les première et seconde structures de montage espacées présentent des première et seconde ouvertures formées dans la partie de corps centrale dimensionnée pour recevoir un goujon (214).

3. Système de collecte d'urine selon la revendication 1, comprenant en outre un tube de vidange (14) en communication de fluide avec l'entrée de fluide ; et
un orifice d'échantillonnage (16) supporté en communication de fluide avec le tube de vidange en amont de l'entrée de fluide.

4. Système de collecte d'urine selon la revendication 3, dans lequel l'orifice d'échantillonnage comprend une extrémité d'entrée (28) conçue pour être en communication de fluide avec un cathéter urinaire et une extrémité de sortie (30) conçue pour être en communication de fluide avec le tube de vidange.

5. Système de collecte d'urine selon la revendication 4, dans lequel l'orifice d'échantillonnage est conçu pour entrer en prise avec une seringue du type à bout coulissant ou à verrou LUER.

6. Système de collecte d'urine selon la revendication 4, dans lequel l'orifice d'échantillonnage comprend un élément flexible et perçable (152) d'obturation étanche.

7. Système de collecte d'urine selon la revendication 3, comprenant en outre un évent en ligne (80) positionné en communication de fluide avec le tube de vidange en amont de l'entrée de fluide de la poche de collecte.
